(19) Europäisches Patentamt
European Patent Office
Office européen des brevets

(11) Veröffentlichungsnummer: **0 005 501**
**B1**

(12) # EUROPÄISCHE PATENTSCHRIFT

(45) Veröffentlichungstag der Patentschrift: **25.11.81**

(21) Anmeldenummer: **79101399.8**

(22) Anmeldetag: **08.05.79**

(51) Int. Cl.³: **C 07 D 263/58,** C 07 D 277/68, C 07 D 417/12, C 07 D 413/12, C 07 D 493/10, C 07 D 513/04, A 01 N 43/74

(54) **Heteroaryl-oxyessigsäureamide, Verfahren zu ihrer Herstellung und ihre Verwendung als Herbizide.**

(30) Priorität: **20.05.78 DE 2822155**
**02.02.79 DE 2903966**

(43) Veröffentlichungstag der Anmeldung: **28.11.79 Patentblatt 79/24**

(45) Bekanntmachung des Hinweises auf die Patenterteilung: **25.11.81 Patentblatt 81/47**

(84) Benannte Vertragsstaaten: **BE CH DE FR GB IT NL**

(56) Entgegenhaltungen:
**CHEMICAL ABSTRACTS, Vol. 77, Nr. 5, 31. Juli 1972, Columbus, Ohio, USA, PIERRE POIGNANT: »Comparative phytocidalactivity of various phenoxyalcanecarboxamides«, Seite 151**
**IL FARMACO EDIZIONE SCIENTIFICA, Band XXIII, Nr. 5, Mai 1979, Pavia, Italien, G. PAGANI et al.: »Arilossi – ed ariltioacetamidi ad attivita fitotossica«, Seiten 332–349**

(73) Patentinhaber: **BAYER AG, Zentralbereich Patente, Marken und Lizenzen, D-5090 Leverkusen 1, Bayerwerk (DE)**

(72) Erfinder: **Förster, Heinz, Dr., Am Eckbusch 47, D-5600 Wuppertal 1 (DE)**
Erfinder: **Hofer, Wolfgang, Dr., Pahlkestrasse 59, D-5600 Wuppertal 1 (DE)**
Erfinder: **Mues, Volker, Dr., Maréestrasse 61, D-5600 Wuppertal 1 (DE)**
Erfinder: **Eue, Ludwig, Dr., Paul-Klee-Strasse 36, D-5090 Leverkusen 1 (DE)**
Erfinder: **Schmidt, Robert R., Dr., Hahnenweg 5, D-5000 Koeln 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 005 501 B1

Heteroaryl-oxyessigsäureamide, Verfahren zu ihrer Herstellung und ihre Verwendung als Herbizide

Die Erfindung betrifft neue Heteroaryl-oxyessigsäureamide, ein Verfahren zu ihrer Herstellung sowie ihre Verwendung als Herbizide.

Es ist bereits bekannt, daß Thiocarbamidsäureester, wie z. B. Hexahydro-1-H-azepin-1-carbamidsäure-thioläthylester (Molinate) und N,N-Diäthyl-thiocarbamidsäure-S-(4-chlorbenzyl)-ester (Benthiocarb), herbizide Eigenschaften besitzen (vgl. US-A-3 198 786 bzw. DE-B-1 817 662). Es ist ferner bekannt, daß Phenoxyalkancarbonsäuren und deren Derivate, wie z. B. 2,4-Dichlorphenoxyessigsäure (2,4-D) und 2,4-Dichlorphenoxypropionsäure (2,4-DP) als Herbizide, speziell als Wuchsstoffherbizide eingesetzt werden können (vgl. z. B. H. Martin, Die wissenschaftlichen Grundlagen des Pflanzenschutzes, Verlag Chemie (1967) S. 426 ff.). Die Wirkung dieser handelsüblichen Produkte ist jedoch nicht immer ganz befriedigend.

Am oder nach dem hierin beanspruchten 1. Prioritätstag (20. 5. 1978) ist außerdem bekannt geworden, daß bestimmte (Hetero)-aryloxy- und (Hetero)-arylthioacetamide herbizide Eigenschaften aufweisen, so z. B. 2-Benzoxazolyl- und 2-Benzimidazolylthioessigsäure-N.N-di-sec.-butylamid [vgl. Il Farmaco, Bd. 23, Heft 5, S. 332–348 (Versanddatum 20. Mai 1978); diese Druckschrift gehört nur bezüglich der durch die Priorität vom 20. 5. 1978 nicht gedeckten Erfindungsteile zum Stand der Technik]. Ein Nachteil dieser Verbindungen ist darin zu sehen, daß sie bei pre-emergence-Anwendung praktisch keine Wirkung gegen grasartige Unkräuter (Monocotyledoneae) besitzen.

Es wurden nun neue Heteroaryl-oxyessigsäureamide der Formel

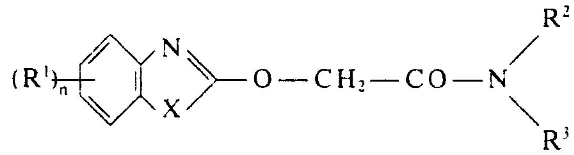

(I)

in welcher

n für 1, 2, 3 oder 4 steht und die Reste

$R^1$ einzeln für Wasserstoff, Halogen, Alkyl mit 1 bis 6 Kohlenstoffatomen, Trifluormethyl, Alkoxy, Alkylthio, jeweils mit 1 bis 6 Kohlenstoffatomen je Alkylrest, Trifluormethoxy, Trifluormethylthio, Nitro oder Cyano steht, oder in denen zwei Reste $R^1$ gemeinsam für die Methylendioxy-, Dichlormethylendioxy- oder Difluormethylendioxygruppe stehen,

$R^2$ und $R^3$, welche gleich oder verschieden sein können, einzeln für geradkettiges oder verzweigtes Alkyl mit 1 bis 10 Kohlenstoffatomen, Cyanoäthyl, 2-Alkoxyäthyl mit 1 bis 5 Kohlenstoffatomen je Alkoxygruppe, Allyl, Propargyl, 1-Methylpropargyl, 1,1-Dimethylpropargyl, Cyclopentyl, Cyclohexyl, Phenyl, Nitrophenyl, Tolyl, Nitrotolyl, Chlorphenyl, Naphthyl, Benzyl, Chlorbenzyl, Chlortolyl oder den Tetrahydrofurfurylrest stehen oder zusammen mit dem Stickstoffatom, an das sie gebunden sind, für Pyrrolidyl, Monoalkyl- oder Dialkylpyrrolidyl mit 1 bis 5 Kohlenstoffatomen je Alkylgruppe, Morpholinyl, Monoalkyl- oder Dialkylmorpholinyl mit 1 bis 5 Kohlenstoffatomen je Alkylgruppe, Piperidyl, Monoalkyl-, Dialkyl- und Trialkyl-piperidyl mit jeweils 1 bis 5 Kohlenstoffatomen je Alkylgruppe, für spirosubstituiertes Piperidyl, für Perhydro-azepinyl (=Hexamethylenimino-Rest), 1,2,3,4-Tetrahydroindolyl, Monoalkyl-, Dialkyl- und Trialkyl-1,2,3,4-tetrahydroindolyl mit jeweils 1 bis 5 Kohlenstoffatomen je Alkylgrupe, Perhydroindolyl, Monoalkyl-, Dialkyl- und Trialkylperhydroindolyl mit jeweils 1 bis 5 Kohlenstoffatomen je Alkylgruppe, 1,2,3,4-Tetrahydro-chinolyl und 1,2,3,4-Tetrahydroiso-chinolyl, Monoalkyl-, Dialkyl- und Trialkyl-1,2,3,4-tetrahydro-chinolyl und -isochinolyl mit jeweils 1 bis 5 Kohlenstoffatomen je Alkylgruppe, Perhydrochinolyl und Perhydroisochinolyl, Monoalkyl-, Dialkyl- und Trialkyl-perhydro-chinolyl und -isochinolyl mit jeweils 1 bis 5 Kohlenstoffatomen je Alkylgruppe, für Perhydrothiazolyl oder den Rest der Formel

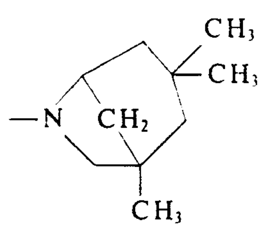

stehen und

X für Sauerstoff oder Schwefel steht,

gefunden.

Man erhält die Heteroaryl-oxyessigsäureamide der Formel (I), wenn man Hydroxyessigsäureamide der Formel

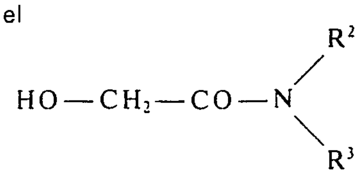

(II)

worin
$R^2$ und $R^3$ die oben angegebene Bedeutung haben,
mit 2-Halogen-benzazolen der Formel

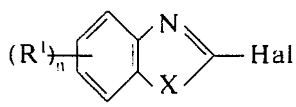

(III)

worin
n, $R^1$ und X die oben angegebene Bedeutung haben und
Hal für Chlor, Fluor, Brom oder Jod steht,
gegebenenfalls in Gegenwart eines Säureakzeptors und gegebenenfalls in Gegenwart eines Verdünnungsmittels umsetzt.

Die neuen Heteroaryl-oxyessigsäureamide (I) zeichnen sich durch starke herbizide Wirksamkeit aus. Überraschenderweise zeigen die erfindungsgemäßen Heteroaryl-oxyessigsäureamide eine wesentlich bessere herbizide Wirkung als die aus dem Stand der Technik bekannten Verbindungen analoger Konstitution und gleicher Wirkungsrichtung. Insbesondere ist es überraschend, daß die erfindungsgemäßen Wirkstoffe eine hervorragende Wirkung gegen Gramineen besitzen, während die konstitutionell ähnlichen Phenoxy-alkancarbonsäure-Derivate, wie z. B. 2,4-D, ebenso wie die seit Mai 1978 bekannten (Hetero)-aryloxy- und (Hetero)-arylthioacetamide, praktisch keine Wirkung gegen Gramineen aufweisen.

Die herbizide Wirkung der erfindungsgemäßen Verbindungen beruht daher offenbar auf einem Mechanismus, der von dem Wirkungsmechanismus der bekannten ähnlichen Verbindungen verschieden ist und der eine unterschiedliche Selektivität und Verwendbarkeit der beanspruchten Wirkstoffe ermöglicht. Wie sich gezeigt hat, sind die neuen Wirkstoffe der Formel (I) im Gegensatz zu den bekannten Phenoxy- und Naphthoxyessigsäuren und -propionsäuren und deren Derivaten, wie insbesondere Salzen, Estern und Amiden, keine Wuchsstoffe. Ferner sind die neuen Wirkstoffe, wiederum im Gegensatz zu den bekannten Herbiziden vom 2,4-D-Typ, nur in Form der anspruchsgemäßen N.N-disubstituierten Glykolamide wirksam, nicht aber in Form der zugehörigen freien Säuren und deren Ester sowie entsprechender Propionsäurederivate, auch nicht in Form der entsprechenden N-unsubstituierten und N-monosubstituierten Amide. Die beanspruchten neuen Wirkstoffe der Formel (I) bilden eine neuartige Gruppe von Gräserherbiziden.

Als Beispiele für die erfindungsgemäßen Heteroaryl-oxyessigsäureamide der Formel (I) seien im einzelnen folgende Verbindungen genannt:

Benzthiazol(2)yloxy- und Benzoxazol(2)yloxy-essigsäure-, -dimethylamid, -diäthylamid, -di-n-propyl-amid, -di-iso-propyl-amid, -N-methyl-N-iso-propylamid, -N-methyl-N-iso-butylamid, -N-methyl-N-sek.-butylamid, -di-(2-äthyl-hexyl)-amid, -N-methyl-N-(2-cyano-äthyl)-amid, -di-(2-methoxy-äthyl)-amid, -diallylamid, -N-methyl-N-propargylamid, -N-methyl-N-(1-methyl-propargyl)-amid, -dipropargylamid, -N-methyl-N-cyclopentylamid, -N-methyl-N-cyclohexylamid, -N-methyl-anilid, -N-methyl-N-(2-nitrophenyl)-, -N-methyl-N-(3-nitrophenyl)-, -N-methyl-N-(4-nitrophenyl)-amid, -N-methyl-N-(2-chlorphenyl)-, -N-methyl-N-(3-chlorphenyl)-, -N-methyl-N-(4-chlorphenyl)-amid, -N-methyl-N-(3-nitro-6-methyl-phenyl)-amid, -N-äthyl-anilid, -N-äthyl-N-(2-nitro-phenyl)-, -N-äthyl-N-(3-nitro-phenyl)-, -N-äthyl-N-(4-nitro-phenyl)-amid, -N-äthyl-N-(2-chlor-phenyl)-, -N-äthyl-N-(3-chlor-phenyl)-, -N-äthyl-N-(4-chlor-phenyl)-amid, -N-äthyl-N-(3-nitro-6-methyl-phenyl)-amid, -N-propyl-anilid, -N-propyl-N-(2-nitro-phenyl)-, -N-propyl-N-(3-nitro-phenyl)-, -N-propyl-N-(4-nitro-phenyl)-amid, -N-propyl-N-(2-chlor-phenyl)-, -N-propyl-N-(3-chlor-phenyl)-, -N-propyl-N-(4-chlor-phenyl)-amid, -N-propyl-N-(2-methyl-phenyl)-, -N-propyl-N-(3-methyl-phenyl)-, -N-propyl-N-(4-methyl-phenyl)-amid, -N-propyl-N-(3-nitro-6-methyl-phenyl)-amid, -N-butyl-anilid, -N-butyl-N-(2-nitrophenyl)-, -N-butyl-N-(3-nitro-phenyl)-, -N-butyl-N-(4-nitro-phenyl)-amid, -N-butyl-N-(2-chlor-phenyl)-, -N-butyl-N-(3-chlor-phenyl)-, -N-butyl-N-(4-chlor-phenyl)-amid, -N-butyl-N-(2-methyl-phenyl)-, -N-butyl-N-(3-methyl-phenyl)-, -N-butyl-N-(4-methyl-phenyl)-amid,

-N-butyl-N-(3-nitro-6-methyl-phenyl)-amid, -N-isobutyl-anilid, -N-iso-butyl-N-(2-nitro-phenyl)-, -N-iso-butyl-N-(3-nitro-phenyl)-, -N-iso-butyl-N-(4-nitro-phenyl)-amid, -N-iso-butyl-N-2-chlor-phenyl), -N-iso-butyl-N-(3-chlor-phenyl)-, -N-iso-butyl-N-(4-chlor-phenyl)-amid, -N-iso-butyl-N-(2-methyl-phenyl)-, -N-iso-butyl-N-(3-methyl-phenyl)-, -N-iso-butyl-N-(4-methyl-phenyl)-amid, -N-iso-butyl-N-(3-nitro-6-methyl-phenyl)-amid, -N-methyl-N-naphth(1)ylamid, -N-methyl-N-naphth(2)ylamid, -N-äthyl-N-naphth(1)ylamid, -N-äthyl-N-naphth(2)ylamid, -N-n-propyl-N-naphth(2)ylamid, -N-iso-propyl-N-naphth(2)ylamid, -N-n-butyl-N-naphth(2)ylamid, -N-iso-butyl-N-naphth(2)ylamid, -dibenzylamid, -N-methyl-N-benzylamid, -N-äthyl-N-benzylamid, -N-propyl-N-benzylamid, -N-butyl-N-benzyl-amid, -pyrrolidid, -2-methyl-pyrrolidid, -morpholid, -piperidid, -2-methyl-piperidid, -4-methyl-piperidid, -2,4-dimethyl-piperidid, -2,4,6-trimethyl-piperidid, -2-äthyl-piperidid, -4-äthyl-piperidid, -2,4-diäthyl-piperidid, -2,4,6-triäthyl-piperidid, -2-methyl-4-äthyl-piperidid, -2-äthyl-4-methyl-piperidid, -2-methyl-5-äthyl-piperidid, -2-äthyl-5-methyl-piperidid, -2-methyl-6-äthyl-piperidid, -1,2,3,4-tetrahydroindolid, -2-methyl-1,2,3,4-tetrahydroindolid, -perhydroindolid, -2-methyl-perhydroindolid, -2,2-dimethyl-perhydroindolid, -1,2,3,4-tetrahydrochinolid, -2-methyl-1,2,3,4-tetrahydrochinolid, -perhydrochinolid, -2-methyl-perhydrochinolid, -1,2,3,4-tetrahydro-iso-chinolid und -perhydroisochinolid.

Verwendet man Hydroxyessigsäure-piperidid und 2-Brombenzthiazol als Ausgangsstoffe, so kann der Reaktionsablauf nach dem erfindungsgemäßen Verfahren durch folgendes Formelschema wiedergegeben werden:

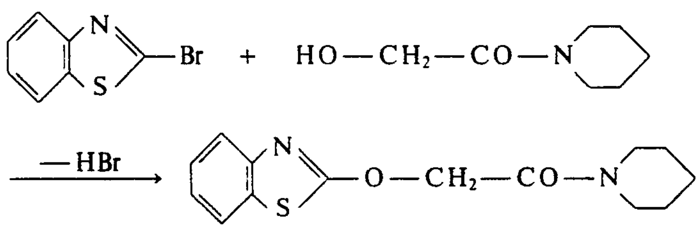


Die als Ausgangsstoffe zu verwendenden Hydroxyessigsäureamide sind durch die Formel (II) allgemein definiert. In dieser Formel stehen $R^2$ und $R^3$ vorzugsweise für diejenigen Reste, die bereits im Rahmen der Substituentendefinitionen der Formel (I) vorzugsweise genannt sind.

Als Ausgangsstoffe der Formel (II) seien beispielsweise die folgenden Hydroxyessigsäureamide genannt:

Hydroxy-essigsäure-, -dimethylamid, -diäthylamid, -di-n-propyl-amid, -di-iso-propyl-amid, -N-methyl-N-iso-propyl-amid, -N-methyl-N-iso-butyl-amid, -N-methyl-N-sek.-butyl-amid, -di-(2-äthyl-hexyl)-amid, -N-methyl-N-(2-cyano-äthyl)-amid, -di-(2-methoxy-äthyl)-amid, -di-allyl-amid, -N-methyl-N-propargyl-amid, -N-methyl-N-(1-methyl-propargyl)-amid, -dipropargyl-amid, -N-methyl-N-cyclopentyl-amid, -N-methyl-N-cyclohexyl-amid, -N-methyl-anilid, -N-methyl-N-(2-nitro-phenyl)-, -N-methyl-N-(3-nitro-phenyl)-, -N-methyl-N-(4-nitro-phenyl)-amid, -N-methyl-N-(2-chlor-phenyl)-, -N-methyl-N-(3-chlor-phenyl)-, -N-methyl-N-(4-chlorphenyl)-amid, -N-methyl-N-(3-nitro-6-methyl-phenyl)-amid, -N-äthyl-anilid, -N-äthyl-N-(2-nitro-phenyl)-, -N-äthyl-N-(3-nitro-phenyl)-, -N-äthyl-N-(4-nitro-phenyl)-amid, -N-äthyl-N-(2-chlor-phenyl)-, -N-äthyl-N-(3-chlor-phenyl)-, -N-äthyl-N-(4-chlor-phenyl)-amid, -N-äthyl-N-(3-nitro-6-methyl-phenyl)-amid, -N-propyl-anilid, -N-propyl-N-(2-nitro-phenyl)-, -N-propyl-N-(3-nitro-phenyl)-, -N-propyl-N-(4-nitro-phenyl)-amid, -N-propyl-N-(2-chlor-phenyl)-, -N-propyl-N-(3-chlor-phenyl)-, -N-propyl-N-(4-chlor-phenyl)-amid, -N-propyl-N-(2-methyl-phenyl)-, -N-propyl-N-(3-methyl-phenyl)-, -N-propyl-N-(4-methyl-phenyl)-amid, -N-propyl-N-(3-nitro-6-methyl-phenyl)-amid, -N-butyl-anilid, -N-butyl-N-(2-nitro-phenyl)-, -N-butyl-N-(3-nitro-phenyl)-, -N-butyl-N-(4-nitro-phenyl)-amid, -N-butyl-N-(2-chlor-phenyl)-, -N-butyl-N-(3-chlor-phenyl)-, -N-butyl-N-(4-chlor-phenyl)-amid, -N-butyl-N-(2-methyl-phenyl)-, -N-butyl-N-(3-methyl-phenyl)-, -N-butyl-N-(4-methyl-phenyl)-amid, -N-butyl-N-(3-nitro-6-methyl-phenyl)-amid, -N-isobutyl-anilid, -N-iso-butyl-N-(2-nitro-phenyl)-, -N-iso-butyl-N-(3-nitro-phenyl)-, -N-iso-butyl-N-(4-nitro-phenyl)-amid, -N-iso-butyl-N-(2-chlor-phenyl)-, -N-iso-butyl-N-(3-chlor-phenyl)-, -N-iso-butyl-N-(4-chlor-phenyl)-amid, -N-iso-butyl-N-(2-methyl-phenyl)-, -N-iso-butyl-N-(3-methyl-phenyl)-, -N-iso-butyl-N-(4-methyl-phenyl)-amid, -N-iso-butyl-N-(3-nitro-6-methyl-phenyl)-amid,

-naphth(1)ylamid, -naphth(2)ylamid, -N-methyl-N-naphth(1)ylamid, -N-methyl-N-naphth(2)ylamid, -N-äthyl-N-naphth(1)ylamid, -N-äthyl-N-naphth(2)ylamid, -N-n-propyl-N-naphth(2)ylamid, -N-iso-propyl-N-naphth(2)ylamid, -N-n-butyl-N-naphth(2)ylamid, -N-iso-butyl-N-naphth(2)ylamid, -dibenzylamid, -N-methyl-N-benzylamid, -N-äthyl-N-benzyl-amid, -N-propyl-N-benzyl-amid, -N-butyl-N-benzyl-amid, -pyrrolidid, -2-methyl-pyrrolidid, -morpholid, -piperidid, -2-methyl-piperidid, -4-methyl-piperidid, -2,4-dimethyl-piperidid, -2,4,6-trimethyl-piperidid, -2-äthyl-piperidid, -4-äthyl-piperidid, -2,4-diäthyl-piperidid, -2,4,6-triäthyl-piperidid, -2-methyl-4-äthyl-piperidid, -2-äthyl-4-methyl-piperidid, -2-methyl-5-äthyl-piperidid, -2-äthyl-5-methyl-piperidid, -2-methyl-6-äthyl-piperidid, -1,2,3,4-tetrahydroindolid, -2-methyl-1,2,3,4-tetrahydroindolid, -perhydroindolid, -2-methyl-perhydroindolid, -2,2-dimethyl-perhydroindolid, -1,2,3,4-tetrahydrochinolid, -2-methyl-1,2,3,4-tetrahydrochinolid, -perhydrochinolid, -2-methyl-perhydrochinolid, -1,2,3,4-tetrahydro-isochinolid und -perhydro-isochinolid.

Die Hydroxyessigsäureamide der Formel (II) sind teilweise bekannt (vgl. DE-A-2 647 481). Sie können, wie in folgendem Schema skizziert, aus den entsprechenden Hydroxyessigsäuren hergestellt werden:

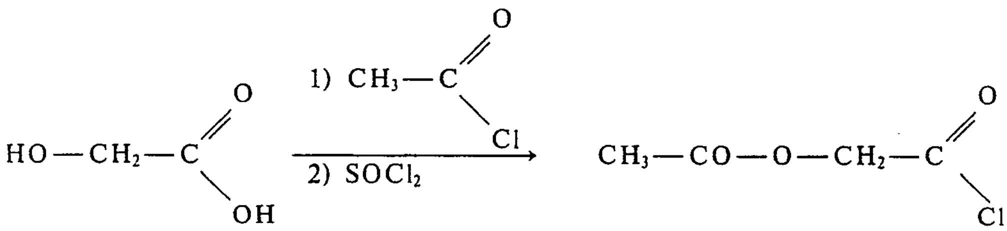


(IV) (V)

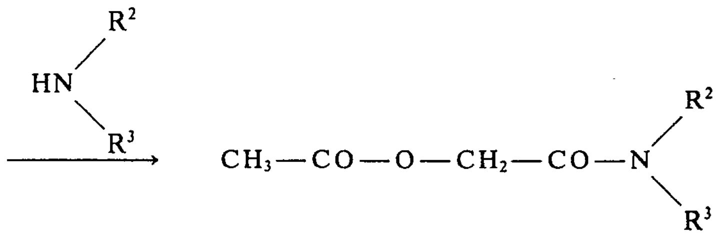

(VI)

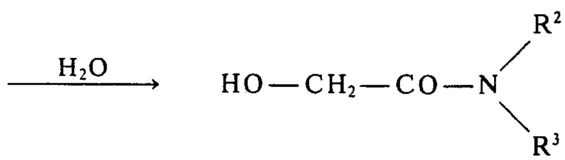


(II)

Hierzu wird zunächst die Hydroxyessigsäure (Glykolsäure) der Formel (IV) mit Acylierungsmitteln, wie z. B. Acetylchlorid, bei Temperaturen zwischen 0 und 50°C, vorzugsweise zwischen 20 und 30°C, umgesetzt und anschließend die resultierenden Reaktionsgemische mit Chlorierungsmitteln, wie z. B. Thionylchlorid, bei Temperaturen zwischen 20 und 100°C, vorzugsweise zwischen 40 und 90°C, behandelt.

Die hierbei erhaltenen Produkte, wie z. B. (V), welche durch Destillation gereinigt werden, können auf konventionelle Weise durch Umsetzung mit den entsprechenden Aminen, gegebenenfalls in Gegenwart inerter Verdünnungsmittel, bei Temperaturen zwischen −10 und +50°C, vorzugsweise zwischen 0 und +30°C, in die Säureamide der Formel (VI) überführt werden. Die Aufarbeitung und Reinigung dieser Produkte erfolgt nach üblichen Methoden durch Waschen mit Wasser, Trocknen der organischen Phase, Abdestillieren des Lösungsmittels und gegebenenfalls Umkristallisation. Durch Umsetzung mit wäßrig-alkoholischer Natronlauge bei Temperaturen zwischen 0 und 50°C, vorzugsweise zwischen 10 und 40°C, werden die Verbindungen der Formel (VI) zu den Verbindungen der Formel (II) entacyliert. Zur Isolierung und Reinigung der Produkte werden die Lösungsmittel im Vakuum abdestilliert, der Rückstand wird mit einem organischen Lösungsmittel wie z. B. Methylenchlorid extrahiert, die Lösung wird getrocknet und das Lösungsmittel abdestilliert. Die Reinigung kann z. B. durch Umkristallisation erfolgen.

Die weiter als Ausgangsstoffe zu verwendenden 2-Halogenbenzazole sind durch Formel (III) definiert.

Vorzugsweise steht darin $R^1$ für diejenigen Reste, die bereits im Rahmen der Substituentendefinitionen der Formel (I) vorzugsweise genannt sind und Hal steht vorzugsweise für Chlor oder Brom; n steht vorzugsweise für 1, 2, 3 oder 4.

Als Ausgangsstoffe der Formel (III) seien beispielsweise genannt: 2-Chlor- und 2-Brom-benzthiazol sowie 2-Chlor- und 2-Brom-benzoxazol.

Die Benzazole der Formel (III) sind bekannte Verbindungen (vgl. Am. Chem. Journal 21, S. 111–167 (1899); J. Prakt. Chem. [2] 42, S. 445–457 (1890); DE-OS 1 164 413 und GB-PS 913 910).

Das Verfahren zur Herstellung der erfindungsgemäßen Heteroaryl-oxyessigsäureamide der Formel (I) wird bevorzugt unter Verwendung geeigneter Lösungs- oder Verdünnungsmittel durchgeführt. Als solche kommen praktisch alle inerten organischen Solventien in Frage. Hierzu gehören insbesondere Alkohole wie Methanol, Äthanol, n- und iso-Propanol, Äther wie Diäthyl- und Dibutyläther, Tetrahydrofuran und Dioxan, Ketone wie Aceton, Methyläthyl-, Methyl-isopropyl- und Methyl-isobutyl-keton, Nitrile wie Acetonitril und Propionitril sowie die hochpolaren Lösungsmittel Dimethylformamid, Dimethylsulfoxid, Sulfolan und Hexamethylphosphorsäuretriamid.

Als Säureakzeptoren können bei der Durchführung des erfindungsgemäßen Verfahrens alle üblicherweise verwendbaren Säurebindemittel eingesetzt werden. Hierzu gehören insbesondere Alkalihydroxide wie Natriumhydroxid und Kaliumhydroxid, Alkalicarbonate wie Natriumcarbonat und -hydrogencarbonat, Kaliumcarbonat und -hydrogencarbonat, Alkalialkoholate wie Natriummethylat und -äthylat, Kaliummethylat und -äthylat, ferner aliphatische, aromatische oder heterocyclische Amine wie z. B. Triäthylamin, Dimethylanilin, Dimethylbenzylamin, Pyridin und Diazabicyclooctan.

Die Reaktionstemperatur kann innerhalb eines größeren Bereichs variiert werden. Im allgemeinen arbeitet man zwischen 0 und 80°C, vorzugsweise bei 10 bis 50°C. Die Umsetzung wird im allgemeinen bei Normaldruck durchgeführt.

Bei der Durchführung des erfindungsgemäßen Verfahrens setzt man je Mol Benzazol der Formel (III) 1,0 bis 1,2 Mol Hydroxyessigsäureamid der Formel (II) und 1,0 bis 1,5 Moläquivalente eines Säureakzeptors ein. Die Umsetzung wird im allgemeinen in einem geeigneten Verdünnungsmittel durchgeführt und das Reaktionsgemisch wird bei der erforderlichen Temperatur mehrere Stunden gerührt.

Die Isolierung der Produkte erfolgt nach üblichen Methoden. Im allgemeinen wird die Reaktionsmischung in Wasser gegossen und mit einer Säure wie z. B. Essigsäure neutralisiert, wobei die Reaktionsprodukte in der Regel kristallin anfallen. Man saugt ab und reinigt die Produkte gegebenenfalls durch Umkristallisation.

Die erfindungsgemäßen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z. B. bei den folgenden Pflanzen verwendet werden:

Dikotyle Unkräuter der Gattungen:

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dicotyle Kulturen der Gattungen:

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cuburbita.

Monokotyle Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea Dactyloctenium, Agrostis, Alopecurus, Apera.

Monokotyle Kulturen der Gattungen:

Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z. B. auf Industrie- und Gleisanlagen und auf Wegen und Plätzen mit und ohne Baumbewuchs. Ebenso können die Verbindungen zur Unkrautbekämpfung in Dauerkulturen z. B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe zeigen insbesondere neben einer sehr guten Wirkung gegen grasartige Unkräuter auch eine gute herbizide Wirkung bei breitblättrigen Unkräutern. Ein selektiver Einatz der erfindungsgemäßen Wirkstoffe ist möglich, vorzugsweise in Rüben, Sojabohnen, Baumwolle, Reis und anderen Getreidearten.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoff-imprägnierte Natur- und synthetische Stoffe, Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z. B. Erdölfraktionen, Alkohole, wie Butanol oder Glycol sowie deren Äther und Ester, Ketone wie Aceton, Methyläthylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z. B. nichtionogene und anionische Emulgatoren, wie Polyoxyäthylen-Fettsäure-Ester, Polyoxyäthylen-Fettalkohol-Äther, z. B. Alkylarylpolyglykol-äther, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können als solche oder in ihren Formulierungen auch in Mischung mit bekannten Herbiziden zur Unkrautbekämpfung Verwendung finden, wobei Fertigformulierung oder Tankmischung möglich ist. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Die Wirkstoffe können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z. B. durch Gießen, Spritzen, Sprühen, Streuen oder Stäuben.

Die erfindungsgemäßen Wirkstoffe können sowohl vor als auch nach dem Auflaufen der Pflanzen appliziert werden. Die Anwendung wird vorzugsweise vor dem Auflaufen der Pflanzen, also im preemergence-Verfahren, vorgenommen. Sie können auch vor der Saat in den Boden eingearbeitet werden.

Die aufgewandte Wirkstoffmenge kann in größeren Bereichen schwanken. Sie hängt im wesentlichen von der Art des gewünschten Effekts ab. Im allgemeinen liegen die Aufwandmengen zwischen 0,1 und 10 kg Wirkstoff pro ha, vorzugsweise zwischen 0,1 und 5 kg/ha.

Die erfindungsgemäßen Wirkstoffe weisen zum Teil bei bestimmten Anwendungskonzentrationen auch eine fungizide Wirkung auf, z. B. gegen den Pilz Pyricularia oryzae, der besonders in Reiskulturen auftritt.

Die nachfolgenden Beispiele dienen zur Erläuterung der Erfindung.

## Herstellungsbeispiele

### Beispiel 1

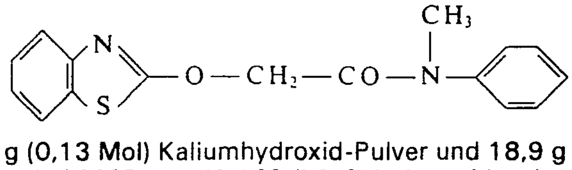

Zu einer Lösung von 7,5 g (0,13 Mol) Kaliumhydroxid-Pulver und 18,9 g (0,11 Mol) Hydroxyessigsäure-N-methylanilid werden bei 20°C 17 g (0,1 Mol) 2-Chlorbenzthiazol gegeben. Die Mischung wird

zwei Stunden bei 40°C gerührt. Zur Aufarbeitung gießt man das Reaktionsgemisch in Wasser und neutralisiert mit Essigsäure. Nach Kristallisation des Produktes wird abgesaugt, einmal mit Wasser und einmal mit Ligroin gewaschen und getrocknet.

Ausbeute: 25 g (84% der Theorie) Benzthiazol(2)yloxyessigsäure-N-methylanilid, weiße Kristalle vom Schmelzpunkt: 118°C.

Analog Beispiel 1 können die folgenden Verbindungen der Formel (Ia)

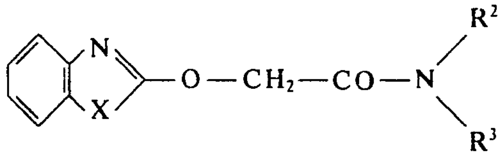

(Ia)

hergestellt werden:

Tabelle 1

| Beispiel Nr. | 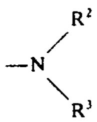 | X | Brechungsindex ($n_D$), Schmelzpunkt (°C) |
|---|---|---|---|
| 2 | 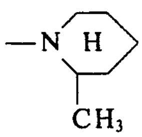 | S | 81 |
| 3 | 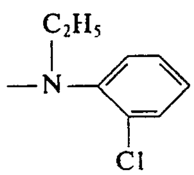 | S | 121 |
| 4 | 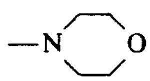 | S | 155 |
| 5 | 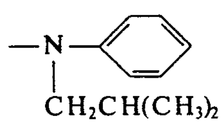 | S | 93 |
| 6 | 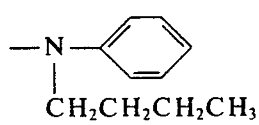 | S | 79 |
| 7 | 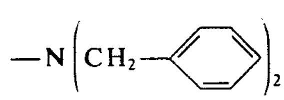 | S | 114 |
| 8 | $-N(CH(CH_3)_2)_2$ | S | 94 |
| 9 | 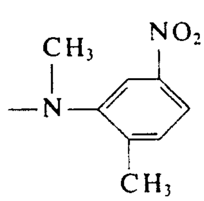 | S | 155 |

(Fortsetzung)

| Beispiel Nr. | 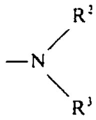 | X | Brechungsindex ($n_D^{20}$); Schmelzpunkt (°C) |
|---|---|---|---|
| 10 | 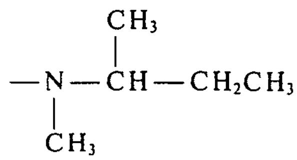 | S | 1,5742 |
| 11 | $-N(CH_2CH_2OCH_3)_2$ | S | 1,5821 |
| 12 | 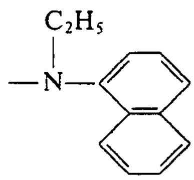 | S | 172 |
| 13 | 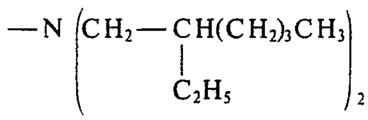 | S | 1,5263 |
| 14 | 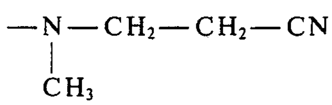 | S | 90 |
| 15 | 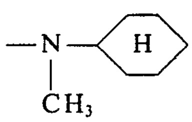 | S | 1,5495 |
| 16 | 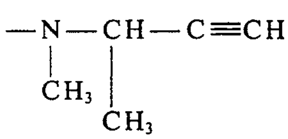 | S | 106 |
| 17 | 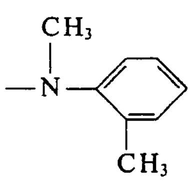 | S | 124 |
| 18 | 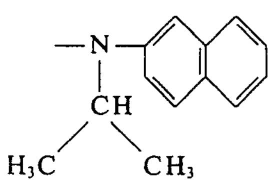 | S | 161 |
| 19 | 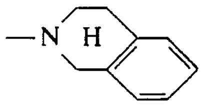 | S | 104 |
| 20 | 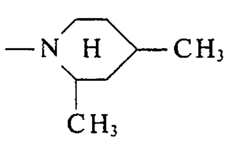 | S | 1,5800 |

(Fortsetzung)

| Beispiel Nr. | 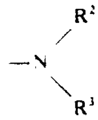 | X | Brechungsindex ($n_D^{20}$), Schmelzpunkt (°C) |
|---|---|---|---|
| 21 | 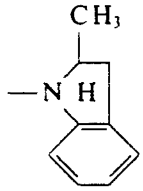 | S | 102 |
| 22 | 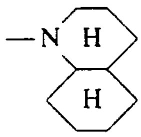 | S | 1,5422 |
| 23 | 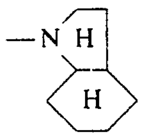 | S | 95 |
| 24 | 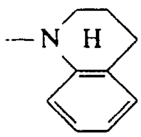 | S | 1,6363 |
| 25 | 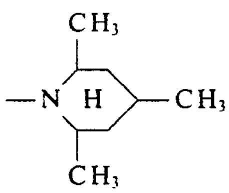 | S | 1,5370 |
| 26 | 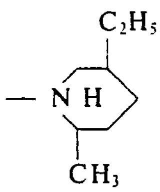 | S | 1,5651 |
| 27 | 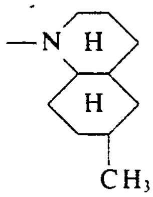 | S | 1,5776 |
| 28 | 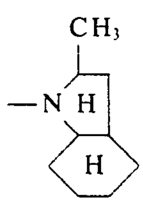 | S | 107 |

(Fortsetzung)

| Beispiel Nr | 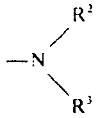 | X | Brechungsindex ($n_D^{20}$); Schmelzpunkt (°C) |
|---|---|---|---|
| 29 | 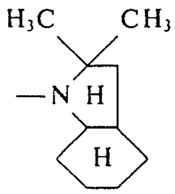 | S | 1,5775 |
| 30 | 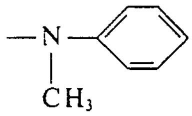 | O | 162 |
| 31 | 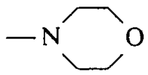 | O | 132 |
| 32 | 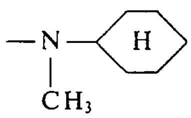 | O | 140 |
| 33 | 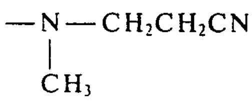 | O | 82 |
| 34 | 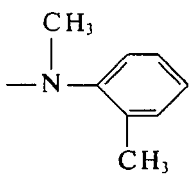 | O | 140 |
| 35 | 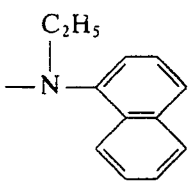 | O | 161–163 |
| 36 | $-N(CH_2CH_2OCH_3)_2$ | O | 88 |
| 37 | 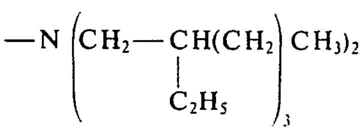 | O | 66 |
| 38 | 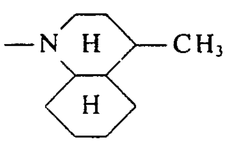 | S | 1,5766 ($n_D$[illegible]) |
| 39 | $-N(CH_3)_2$ | S | 129 |
| 40 | 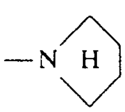 | S | 116 |
| 41 | $-N(C_2H_5)_2$ | S | 1,5860 |

(Fortsetzung)

| Beispiel Nr. | 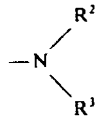 | X | Brechungsindex ($n_D$), Schmelzpunkt (°C) |
|---|---|---|---|
| 42 | 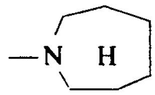 | S | 1,5860 |
| 43 | 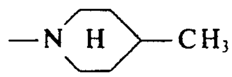 | S | 82 |
| 44 | $—N(CH_2CH{=}CH_2)_2$ | S | 1,5781 |
| 45 | 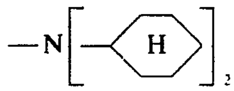 | S | 112 |
| 46 | 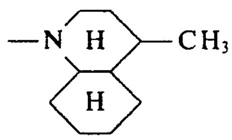 | S | $n_D^{23}$: 1,5766 |
| 47 | 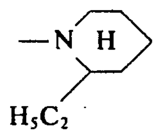 | S | $n_D^{22}$: 1,5730 |
| 48 | 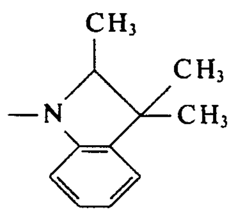 | S | $n_D^{22,5}$: 1,5225 |
| 49 | 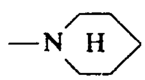 | S | $n_D^{21}$: 1,6173 |
| 50 | 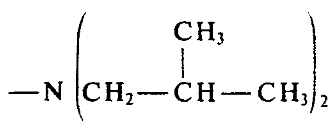 | S | 58 |
| 51 | 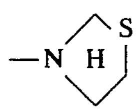 | S | 130 |
| 52 | 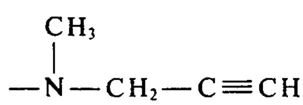 | S | 108 |
| 53 | 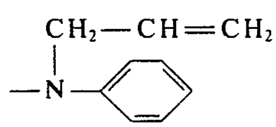 | S | 111 |
| 54 | 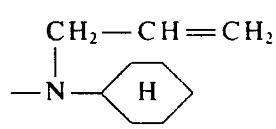 | S | 78 |

(Fortsetzung)

| Beispiel Nr | 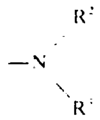 | X | Brechungsindex ($n_D^{20}$); Schmelzpunkt (°C) |
|---|---|---|---|
| 55 | 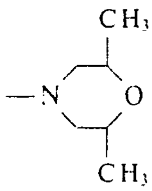 | S | 164 |
| 56 | 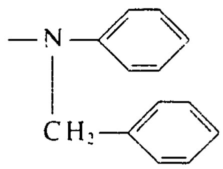 | S | 118 |
| 57 | 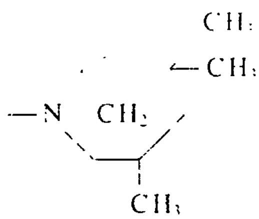 | S | 139 |
| 58 | 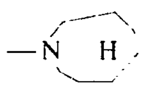 | S | 110 |
| 59 | 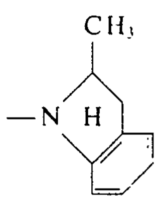 | S | 122 |
| 60 | 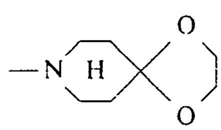 | S | 124 |
| 61 | 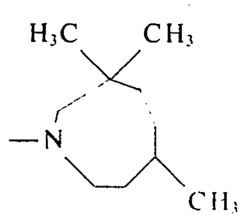 | S | 103 |
| 62 | 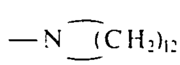 | S | 90 |
| 63 | 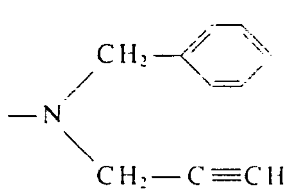 | S | 90 |

(Fortsetzung)

| Beispiel Nr. | 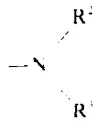 | X | Brechungsindex ($n_D^{20}$) Schmelzpunkt (°C) |
|---|---|---|---|
| 64 | 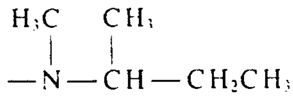 | O | $n_D^{20}$: 1,5983 |
| 65 | 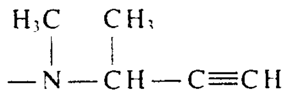 | O | 93 |
| 66 | 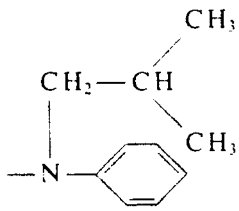 | O | $n_D^{20}$: 1,5616 |
| 67 | 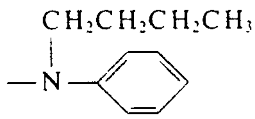 | O | 87 |
| 68 | 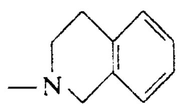 | O | 110 |
| 69 | $-N(CH_2CH{=}CH_2)_2$ | O | $n_D^{20}$: 1,5495 |
| 70 | 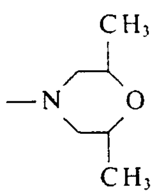 | O | 118 |
| 71 | 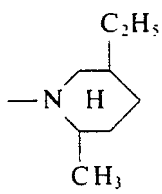 | O | $n_D^{20}$: 1,5425 |
| 72 | 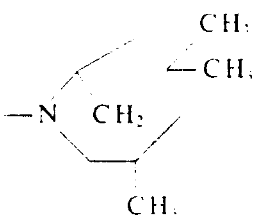 | O | 142 |
| 73 | 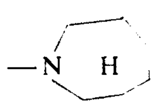 | O | 100 |

(Fortsetzung)

| Beispiel Nr | 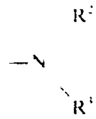 | X | Brechungsindex ($n_D^{20}$); Schmelzpunkt (°C) |
|---|---|---|---|
| 74 | 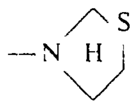 | O | 94–96 |
| 75 | 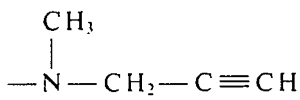 | O | 80–82 |
| 76 | 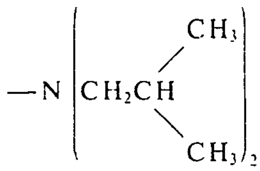 | O | 93 |
| 77 | 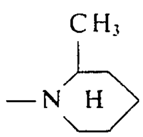 | O | $n_D^{24}$: 1,5560 |
| 78 | 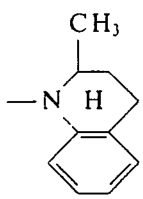 | O | 122 |
| 79 | 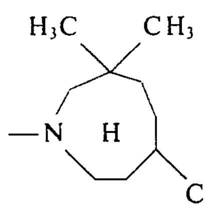 | O | 109 |
| 80 | 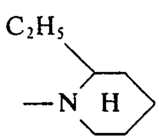 | O | $n_D^{21}$: 1,5440 |
| 81 | 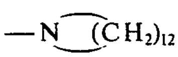 | O | 148 |
| 82 | 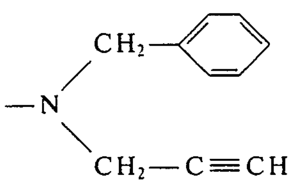 | O | 83 |

Analog Beispiel 1 können auch die folgenden Verbindungen der Formel (I)

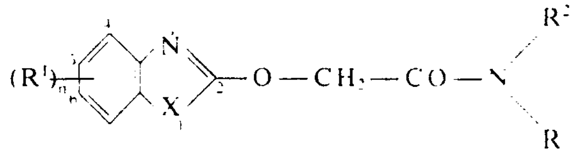

(I)

hergestellt werden.

Tabelle 2

| Beispiel Nr | n | $R^1$ | $R^2$ \ $R^3$ | X | [illegible] |
|---|---|---|---|---|---|
| 83 | 2 | 4,6-Cl | 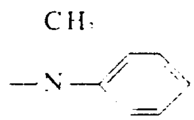 | O | 116 |
| 84 | 1 | 6-CF | 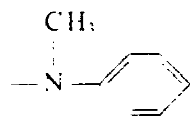 | S | 126 |
| 85 | 1 | 5-$OCF_3$ | 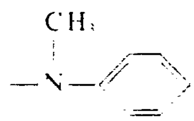 | S | 84 |
| 86 | 2 | 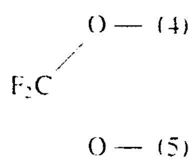 | 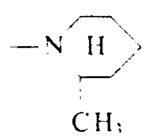 | S | 93 |
| 87 | 1 | 6-$OC_2H_5$ | 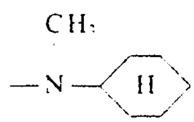 | S | 124-132 |
| 88 | 1 | 6-$OC_2H_5$ | 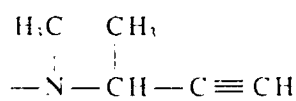 | S | 129 |
| 89 | 1 | 6-$OC_2H_5$ | 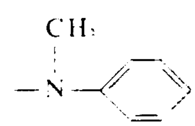 | S | 137-139 |
| 90 | 1 | 6-$OC_2H_5$ | $-N(CH_2CH_2OCH_3)_2$ | S | 1,5623 |
| 91 | 3 | 4,6,7-Cl | 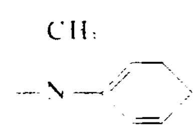 | S | 83 |
| 92 | 2 | 4,6-I | 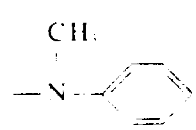 | S | 88 |

(Fortsetzung)

| Beispiel Nr | n | R | $R^2$<br>-N<br>$R^3$ | X | Brechungsindex ($n_D^{20}$)<br>Schmelzpunkt (°C) |
|---|---|---|---|---|---|
| 93 | 4 | H | 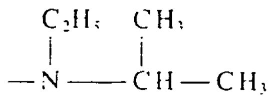 | S | |
| 94 | 4 | H | 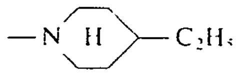 | S | |
| 95 | 1 | 5-Cl | 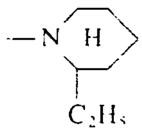 | O | |
| 96 | 1 | 5-Cl | 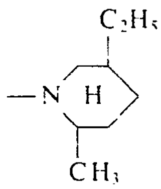 | O | |
| 97 | 1 | 5-Cl | 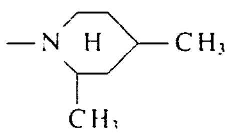 | O | |
| 98 | 1 | 5-Cl | 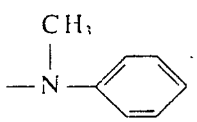 | O | |
| 99 | 1 | $6-OC_2H_5$ | 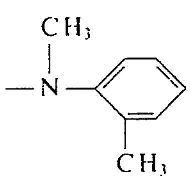 | S | |
| 100 | 1 | $6-OC_2H_5$ | 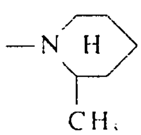 | S | |
| 101 | 4 | H | 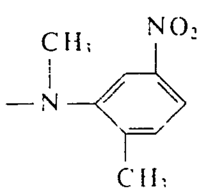 | O | |
| 102 | 4 | H | $-N(C_2H_5)_2$ | O | |
| 103 | 4 | H | 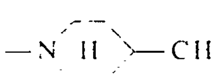 | O | |
| 104 | 4 | H | $-N(CH_3)_2$ | O | |

(Fortsetzung)

| Beispiel Nr | n | R | R R | X | [illegible] |
|---|---|---|---|---|---|
| 105 | 4 | H | 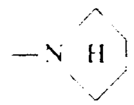 | O | |
| 106 | 4 | H | 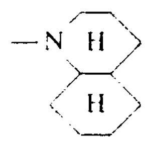 | O | |
| 107 | 4 | H | 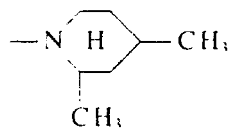 | O | |

Die als Ausgangsverbindungen zu verwendenden Hydroxyessigsäureamide der Formel (II)

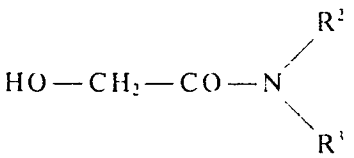 (II)

können beispielsweise wie folgt hergestellt werden:

a) $CH_3-CO-O-CH_2-CO-Cl$

76 g (1 Mol) wasserfreie Hydroxyessigsäure werden bei 20 bis 30°C tropfenweise mit 141 g (1,7 Mol) Acetylchlorid versetzt, wobei eine starke Entwicklung von Chlorwasserstoff zu beobachten ist. Die Mischung wird 3 Stunden auf eine Temperatur von 90 bis 100°C erhitzt; dann wird das überschüssige Acetylchlorid abdestilliert. Man erwärmt den Rückstand auf 30°C und gibt dazu tropfenweise 174 g (1,42 Mol) Thionylchlorid. Die Reaktionsmischung wird 3 Stunden zum Sieden erhitzt. Die Aufarbeitung erfolgt durch Vakuumdestillation.

Ausbeute: 96 g (70% der Theorie) Acetoxyacetylchlorid, farblose Flüssigkeit vom Siedepunkt: 65°C/30 mbar.

b) 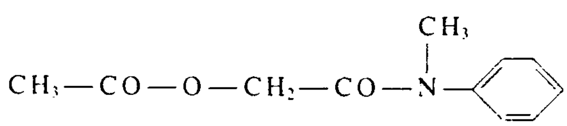

230 g (1,6 Mol) Acetoxyacetylchlorid werden bei 0 bis 10°C zu 377 g (3,5 Mol) N-Methylanilin in 800 ml Toluol gegeben und die Mischung wird ca. 15 Stunden bei 20°C gerührt. Zur Aufarbeitung gießt man die Mischung in Wasser, wäscht die organische Phase mit verdünnter Salzsäure und dann mit Wasser, trocknet und engt ein. Dabei kristallisiert das Produkt aus.

Ausbeute: 246 g (74% der Theorie) Acetoxy-N-methylacetanilid, gelbliche Kristalle vom Schmelzpunkt: 91°C.

c) 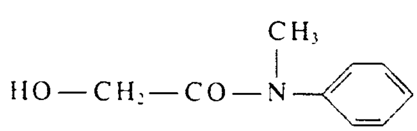

432 g (2,16 Mol) Acetoxy-N-methylacetanilid in 1082 ml Methanol werden bei 30°C mit 197 ml (2,38 Mol) konzentrierter Natronlauge versetzt und die Mischung 15 Stunden bei 20°C gerührt. Dann wird bei 40°C im Vakuum eingeengt und der Rückstand mit Methylenchlorid extrahiert. Die Methylenchloridlösung wird durch Absaugen vom ungelösten Natriumacetat befreit und mit Natriumsulfat

getrocknet. Beim Einengen kristallisiert das Produkt aus.

Ausbeute: 308 g (86% der Theorie) Hydroxyessigsäure-N-methylanilid, blaßgelbe Kristalle vom Schmelzpunkt: 45°C.

Verwendungsbeispiel A

Pre-emergence-Test

Lösungsmittel. 5 Gewichtsteile Aceton
Emulgator: 1 Gewichtsteil Alkylarylpolyglycoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)
100% = totale Vernichtung

Bei diesem Test zeigen z. B. die folgenden Verbindungen der Herstellungsbeispiele eine ausgezeichnete Wirkung: Nr. 1, 2, 16, 17, 20, 24, 30, 32, 34, 36, 44, 47, 64, 65.

**Patentansprüche**

1. Heteroaryl-oxyessigsäureamide der Formel

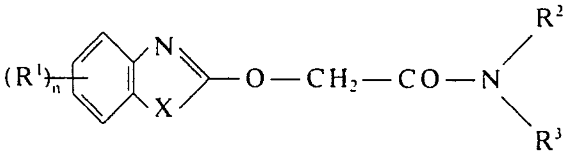

(I)

in welcher

n für 1, 2, 3 oder 4 steht und die Reste

$R^1$ einzeln für Wasserstoff, Halogen, Alkyl mit 1 bis 6 Kohlenstoffatomen, Trifluormethyl, Alkoxy, Alkylthio, jeweils mit 1 bis 6 Kohlenstoffatomen je Alkylrest, Trifluormethoxy, Trifluormethylthio, Nitro oder Cyano steht, oder in denen zwei Reste $R^1$ gemeinsam für die Methylendioxy-, Dichlormethylendioxy- oder Difluormethylendioxygruppe stehen

$R^2$ und $R^3$, welche gleich oder verschieden sein können, einzeln für geradkettiges oder verzweigtes Alkyl mit 1 bis 10 Kohlenstoffatomen, Cyanoäthyl, 2-Alkoxyäthyl mit 1 bis 5 Kohlenstoffatomen je Alkoxygruppe, Allyl, Propargyl, 1-Methylpropargyl, 1,1-Dimethylpropargyl, Cyclopentyl, Cyclohexyl, Phenyl, Nitrophenyl, Tolyl, Nitrotolyl, Chlorphenyl, Naphthyl, Benzyl, Chlorbenzyl, Chlortolyl oder den Tetrahydrofurfurylrest stehen oder zusammen mit dem Stickstoffatom, an das sie gebunden sind, für Pyrrolidyl, Monoalkyl- oder Dialkylpyrrolidyl mit 1 bis 5 Kohlenstoffatomen je Alkylgruppe, Morpholinyl, Monoalkyl- oder Dialkylmorpholinyl mit 1 bis 5 Kohlenstoffatomen je Alkylgruppe, Piperidyl, Monoalkyl-, Dialkyl- und Trialkyl-piperidyl mit jeweils 1 bis 5 Kohlenstoffatomen je Alkylgruppe, für spirosubstituiertes Piperidyl, für Perhydro-azepinyl (=Hexamethylenimino-Rest), 1,2,3,4-Tetrahydroindolyl, Monoalkyl-, Dialkyl- und Trialkyl-1,2,3,4-tetrahydroindolyl mit jeweils 1 bis 5 Kohlenstoffatomen je Alkylgruppe, Perhydroindolyl, Monoalkyl-, Dialkyl- und Trialkylperhydroindolyl mit jeweils 1 bis 5 Kohlenstoffatomen je Alkylgruppe, 1,2,3,4-Tetrahydro-chinolyl und 1,2,3,4-Tetrahydroiso-chinolyl, Monoalkyl-, Dialkyl- und Trialkyl-1,2,3,4-tetrahydro-chinolyl und -isochinolyl mit jeweils 1 bis 5 Kohlenstoffatomen je Alkylgruppe, Perhydrochinolyl und Perhydroisochinolyl, Monoalkyl-, Dialkyl- und Trialkyl-perhydro-chinolyl und -isochinolyl mit jeweils 1 bis 5 Kohlenstoffatomen je Alkylgruppe, für Perhydrothiazolyl oder den Rest der Formel

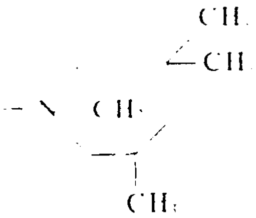

stehen und

X für Sauerstoff oder Schwefel steht.

2. Verfahren zur Herstellung von Heteroaryl-oxyessigsäureamiden der Formel (I) gemäß Anspruch 1, dadurch gekennzeichnet, daß man Hydroxyessigsäureamide der Formel

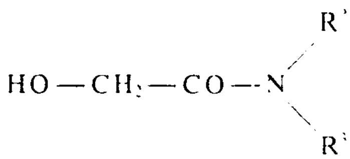 (II)

worin
$R^2$ und $R^3$ die oben angegebene Bedeutung haben,
mit 2-Halogen-benzazolen der Formel

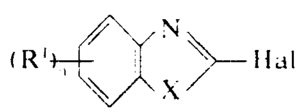 (III)

worin
n, $R^1$ und X die oben angegebene Bedeutung haben und
Hal für Chlor, Fluor, Brom oder Jod steht,
gegebenenfalls in Gegenwart eines Säureakzeptors und gegebenenfalls in Gegenwart eines Verdünnungsmittels umsetzt.

3. Herbizide Mittel, gekennzeichnet durch einen Gehalt an Heteroaryl-oxyessigsäureamiden der Formel (I) gemäß Anspruch 1.

4. Verfahren zur Herstellung von herbiziden Mitteln, dadurch gekennzeichnet, daß man Heteroaryl-oxyessigsäureamide der Formel (I) gemäß Anspruch 1 mit Streckmitteln und/oder oberflächenaktiven Mitteln vermischt.

5. Verwendung von Heteroaryl-oxyessigsäureamiden der Formel (I) gemäß Anspruch 1 zur Bekämpfung von Pflanzenwachstum.

**Claims**

1. Heteroaryl-oxyacetic acid amides of the formula

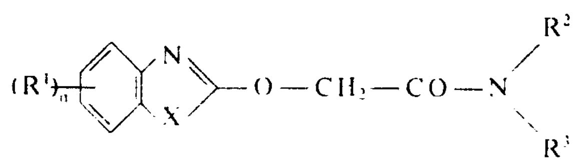 (I)

in which

n represents 1, 2, 3 or 4 and the radicals

$R^1$ individually represent hydrogen, halogen, alkyl with 1 to 6 carbon atoms, trifluoromethyl, alkoxy, alkylthio, in each case with 1 to 6 carbon atoms per alkyl radical, trifluoromethoxy, trifluoromethylthio, nitro or cyano, or in which two radicals $R^1$ together represent the methylenedioxy, dichloromethylenedioxy or difluoromethyledioxy group,

$R^2$ and $R^3$, which can be identical or different, individually represent straight-chain or branched alkyl with 1 to 10 carbon atoms, cyanoethyl, 2-alkoxyethyl with 1 to 5 carbon atoms per alkoxy group, allyl, propargyl, 1-Methylpropargyl, 1,1-dimethylpropargyl, cyclopentyl, cyclohexyl, phenyl, nitrophenyl, tolyl, nitrotolyl, chlorophenyl, naphthyl, benzyl, chlorobenzyl, chlorotolyl or the tetrahydrofurfuryl radical or, together with the nitrogen atom to which they are bonded, represent pyrrolidyl, monoalkyl- or dialkyl-pyrrolidyl with 1 to 5 carbon atoms per alkyl group, morpholinyl, monoalkyl- or dialkyl-morpholinyl with 1 to 5 carbon atoms per alkyl group, piperidyl, monoalkyl-, dialkyl- and trialkyl-piperidyl with in each case 1 to 5 car

bon atoms per alkyl group, spirosubstituted piperidyl, perhydro-azepinyl (=hexamethylene-imino radical), 1,2,3,4-tetrahydroindolyl, monoalkyl-, dialkyl- and trialkyl-1,2,3,4-tetrahydroindolyl with in each case 1 to 5 carbon atoms per alkyl group, perhydroindolyl, monoalkyl-, dialkyl- and trialkyl-perhydroindolyl with in each case 1 to 5 carbon atoms per alkyl group, 1,2,3,4-tetrahydro-quinolyl and 1,2,3,4-tetrahydro-isoquinolyl, monoalkyl-, dialkyl- and trialkyl-1,2,3,4-tetrahydro-quinolyl and -isoquinolyl with in each case 1 to 5 carbon atoms per alkyl group, perhydroquinolyl and perhydroiso-quinolyl, monoalkyl-, dialkyl, and trialkylperhydro-quinolyl and -isoquinolyl with in each case 1 to 5 carbon atoms per alkyl group, perhydrothiazolyl or the radical of the formula

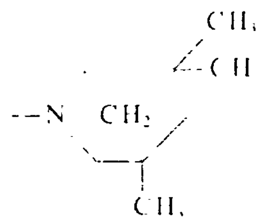

and

X represents oxygen or sulphur.

2. Process for the preparation of heteroaryl-oxyacetic acid of the formula (I) according to Claim 1, characterised in that hydroxy acetic acid amides of the formula

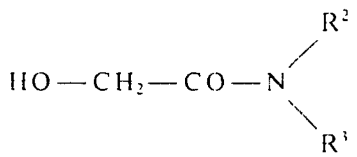

(II)

in which

$R^2$ and $R^3$ have the meaning indicated above,

are reacted with 2-halogeno-benzazoles of the formula

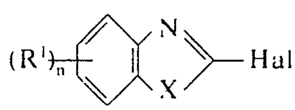

(III)

in which

n, $R^1$ and X have the meaning indicated above and

Hal represents chlorine, fluorine, bromine or iodine,

if appropriate in the presence of an acid acceptor and if appropriate in the presence of a diluent.

3. Herbicidal agents, characterised in that they contain heteroaryl-oxyacetid acid amides of the formula (I) according to Claim 1.

4. Process for the preparation of herbicidal agents, characterised in that heteroaryl-oxyacetic acid amides of the formula (I) according to Claim 1 are mixed with extenders and/or suface-active agents.

5. Use of heteroaryl-oxyacetic acid amides of the formula (I) according to Claim 1 for combating plant growth.

**Revendications**

1. Amides d'acides hétéroaryl-oxyacétiques de formule:

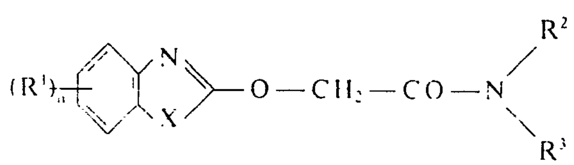

(I)

dans laquelle:

n est égal à 1, 2, 3 ou 4 et les restes

$R^1$ représentent individuellement de d'hydrogène, un halogène, un groupe alkyle ayant 1 à 6 atomes de carbone, le groupe trifluorométhyle, un groupe alkoxy, alkylthio, avec dans chaque cas 1 à 6 atomes de carbone par reste alkyle, le groupe trifluorométhoxy, trifluoro-

méthylthio, nitro ou cyano, ou bien deux restes $R^1$ représentent ensemble le groupe méthylènedioxy, dichlorométhylèndioxy ou difluorométhylènedioxy,

$R^2$ et $R^3$, qui peuvent être égaux ou différents, représentent individuellement un groupe alkyle à chaine droite ou ramifiée ayant 1 à 10 atomes de carbone, le groupe cyanéthyle, un groupe 2-alkoxyéthyle ayant 1 à 5 atomes de carbone par groupe alkoxy, le groupe allyle, propargyle, 1-méthylpropargyle, 1,1-diméthylpropargyle, cyclopentyle, cyclohexyle, phényle, nitrophényle, tolyle, nitrotolyle, chlorophényle, naphthyle, benzyle, chlorobenzyle, chlorotolyle ou le reste tétrahydrofurfuryle, ou forment avec l'atome d'azote auquel ils sont liés, un reste pyrrolidyle, mono-alkyl- ou dialkylpyrrolidyle ayant 1 à 5 atomes de carbone par groupe alkyle, morpholinyle, mono-alkyl- ou dialkylmorplinyle ayant 1 à 5 atomes de carbone par groupe alkyle pipéridyle, mono-alkyl-, dialkyl- et trialkyl-pipéridyle ayant dans chaque cas 1 à 5 atomes de carbone par groupe alkyle, un reste pipéridyle spiro-substitué, le reste perhydro-azépinyle (=reste hexaméthylène-imino), le reste 1,2,3,4-tétrahydro-indolyle, un reste mono-alkyl-, dialkyl- et trialkyl-1,2,3,4-tétrahydro-indolyle ayant dans chaque cas 1 à 5 atomes de carbone par groupe alkyle, le reste perhydro-indolyle, un reste mono-alkyl-, dialkyl- et trialkylperhydro-indolyle ayant dans chaque cas 1 à 5 atomes de carbone par groupe alkyle, le reste 1,2,3,4-tétrahydro-quinoléyle et le reste 1,2,3,4-tétrahydro-isoquinoléyle, un reste mono-alkyl-, dialkyl- et trialkyl-1,2,3,4-tétrahydro-quinoléyle et -isoquinoléyle ayant dans chaque cas 1 à 5 atomes de carbone par groupe alkyle, le reste perhydro-quinoléyle et perhydro-isoquinoléyle, un reste mono-alkyl-, dialkyl- et trialkyl-perhydro-quinoléyle et -isoquinoléyle ayant dans chaque cas 1 à 5 atomes de carbone par groupe alkyle, le reste perhydro-thiazolyle ou le reste de formule:

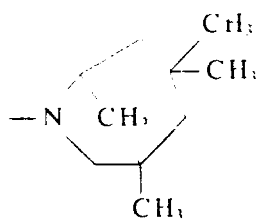

et

X est l'oxygène ou le soufre.

2. Procédé de production d'amides d'acides hétéroaryl-oxyacétiques de formule (I) suivant la revendication 1, caractérisé en ce qu'on fait réagir des amides d'acide hydroxyacétique de formule:

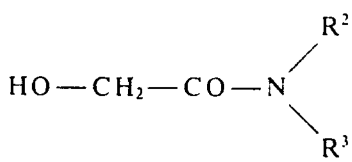

(II)

dans laquelle:

$R^2$ et $R^3$ ont la définition indiquée ci-dessus,

avec des 2-halogéno-benzazoles de formule:

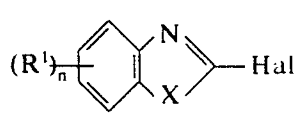

(III)

dans laquelle:

n, $R^1$ et X ont la définition indiquée ci-dessus et

Hal représente le chlore, le fluor, le brome ou l'iode,

éventuellement en présence d'un accepteur d'acide et, éventuellement, en présence d'un diluant.

3. Compositions herbicides, caractérisées par une teneur en amides d'acides hétéroaryl-oxyacétiques de formule (I) suivant la revendication 1.

4. Procédé de production de compositions herbicides, caractérisé en ce qu'on mélange des amides d'acides hétéroaryl-oxyacétiques de formule (I) suivant la revendication 1 avec des diluants et/ou des agents tensioactifs.

5. Utilisation d'amides d'acides hétéroaryl-oxyacétiques de formule (I) suivant la revendiccation 1 pour lutter contre la croissance de plantes.